# EUROPEAN PATENT SPECIFICATION

(11) **EP 0 674 526 B1**
(45) Date of publication and mention of the grant of the patent: **07.05.2003**
(21) Application number: 94904861.5
(22) Date of filing: 16.12.1993
(51) Int. Cl.: A61K 39/00

(54) **VACCINATION WITH PEPTIDE OF MHC CLASS II MOLECULES FOR TREATMENT OF AUTOIMMUNE DISEASE**
IMPFUNG MIT PEPTIDEN VON MHC KLASSE II MOLEKÜLEN ZUR BEHANDLUNG VON AUTOIMMUNKRANKHEITEN
VACCINATION A L'AIDE D'UN PEPTIDE DE MOLECULES DE CLASSE II DU COMPLEXE MAJEUR D'HISTOCOMPATIBILITE (MHC) POUR LE AU TRAITEMENT DE MALADIES AUTOIMMUNES

(30) Priority: 17.12.1992 US 992942
(43) Date of publication of application: 04.10.1995
(73) Proprietor: SRIRAM, Subramaniam, Burlington, VT 05401 (US); NAG, Bishwajit, Pacifica, CA 94044 (US); SHARMA, Somesh D., Los Altos, CA (US)
(72) Inventor: SRIRAM, Subramaniam, Burlington, VT 05401 (US); NAG, Bishwajit, Pacifica, CA 94044 (US); SHARMA, Somesh D., Los Altos, CA (US)
(74) Representative: Greaves, Carol Pauline
(86) International application number: US9312351
(87) International publication number: WO94013320

(56) References cited:
- EP-A- 0 286 447
- WO-A-92/16234
- EUR J IMMUNOL 21 (9). 1991. 2063-2068, XP000608712 ROUDIER J ET AL: "TOLERANCE TO A SELF PEPTIDE FROM THE THIRD HYPERVARIABLE REGION OF THE E-BETA-S CHAIN IMPLICATIONS FOR MOLECULAR MIMICRY MODELS OF AUTOIMMUNE DISEASE."
- PROC NATL ACAD SCI U S A 88 (24). 1991. 11465-11469, XP002017900 SHARMA S D ET AL: "ANTIGEN- SPECIFIC THERAPY OF EXPERIMENTAL ALLERGIC ENCEPHALOMYELITIS BY SOLUBLE CLASS II MAJOR HISTOCOMPATIBILITY COMPLEX-PEPTIDE COMPLEXES."
- Ann. Review Immunol., Vol. 7, issued 1989, H. ACHA-ORBEA et al., "T Cell Receptors in Murine Autoimmune Diseases", pages 371-405, see entire document.
- D.P. STITES et al., "Basic and Clinical Immunology", published 1991 by Appleton and Lange (Conn.), pages 101-108, see entire document.
- Proc. Natl. Acad. Sci., Vol. 78, issued 1981, L. STEINMAN et al., "In Vivo Effects of Antibodies to Immune Response Gene Products: Prevention of Experimental Allergic Encephalitis", pages 7111-7114, see Abstract.
- Advances in Immunology, Vol. 49, issued 1991, L. STEINMAN, "The Development of Rational Strategies for Selective Immunotherapy Against Autoimmune Demyelinating Disease", pages 357-379, see entire document.

## Description

### BACKGROUND OF THE INVENTION

The present invention relates to novel compositions and methods for inhibiting immune responses associated with autoimmune diseases and allergic responses. In particular, it relates to vaccination with peptides from, for instance, the hypervariable region of MHC molecules encoded by alleles associated with disease.

A number of pathological responses involving unwanted immune responses are known. For instance, a number of allergic diseases, have been associated with particular MHC alleles or suspected of having an autoimmune component. Other deleterious T cell-mediated responses include the destruction of foreign cells that are purposely introduced into the body as grafts or transplants from allogeneic hosts. This process, known as "allograft rejection," involves the interaction of host T cells with foreign MHC molecules. Quite often, a broad range of MHC alleles are involved in the response of the host to an allograft.

Autoimmune disease is a particularly important class of deleterious immune response. In autoimmune diseases, self-tolerance is lost and the immune system attacks "self" tissue as if it were a foreign target. More than 30 autoimmune diseases are presently known; these include many which have received much public attention, including myasthenia gravis (MG) and multiple sclerosis (MS).

A crude approach to treating autoimmune disease and other immunopathologies is general immunosuppression. This has the obvious disadvantage of crippling the ability of the subject to respond to real foreign materials to which it needs to mount an immune response. Recent approaches to treating autoimmune disease have involved the use of peptides having an amino acid sequence encoded by a T-cell receptor V gene. The peptides have been proposed as vaccines for preventing, suppressing and treating immune related diseases (see, International Application No. WO 91/01133. Another approach involves the use of monoclonal antibodies against MHC gene products. The antibodies have been proposed for use in targeting cell bearing MHC molecules associated with particular diseases (see, EP Publication No. 68790).

These prior art methods fail to provide a simple self-mediated method for specifically eliminating immune responses restricted by glycoproteins encoded by MHC alleles associated with a variety of deleterious immune responses. Such methods can be used to prevent and/or suppress diseases, particularly those in which the antigen or allergen is not known.

### SUMMARY OF THE INVENTION

The present invention relates to methods and compositions for inhibiting deleterious immune responses. The compositions of the invention comprise an isolated immunogenic MHC polypeptide. The immunogenic MHC polypeptide is usually from a hypervariable region in a Class II molecule. Hypervariable regions from Class II β chains are typically used. The polypeptides are used to induce an immune response against the target sequence of the MHC molecule, thereby rendering the MHC molecules ineffective in initiating the deleterious immune response.

The MHC molecule can be associated with autoimmune disease, such as multiple sclerosis. Alternatively, it may be associated with an allergic response, to a number of allergens, such as ragweed.

The invention also provides pharmaceutical compositions comprising the polypeptides. The compositions can be used for the treatment of autoimmune diseases or allergic responses. The compositions can be administered prophylactically or after the condition has been diagnosed.

### Definitions

The term "peptide" is used interchangeably with "oligopeptide" or "polypeptide" in the present specification to designate a series of residues, typically L-amino acids, connected one to the other typically by peptide bonds between the α-amino and carbonyl groups of adjacent amino acids.

An "immunogenic MHC polypeptide" or of the present invention is a polypeptide capable of eliciting an immune response against an MHC molecule associated with a deleterious immune response in a patient. As set forth in more detail below, the sequence of residues in the polypeptide will be identical to or substantially identical to a polypeptide sequence in the MHC molecule. Thus, a polypeptide of the invention that has a sequence "from a region in an MHC molecule" (*e.g*., the hypervariable region) is polypeptide that has a sequence either identical to or substantially identical to the naturally occurring MHC amino acid sequence of the region. Typically, the polypeptide sequence in the MHC molecule will be from a hypervariable region.

As used herein a "hypervariable region" of an MHC molecule is a region of the molecule in which polypeptides encoded by different alleles at the same locus have high sequence variability or polymorphism. The polymorphism is typically concentrated in the α1 and α2 domains of in Class I molecules and in the α1 and β1 domains of Class II molecules. The number of alleles and degree of polymorphism among alleles may vary at different loci. For instance, in HLA-DR molecules all the polymorphism is attributed to the β chain and the α chain is relatively invariant. For HLA-DQ, both the α and β chains are polymorphic.

The phrases "isolated" or "biologically pure" refer to material which is substantially or essentially free from components which normally accompany it as found in its native state. Thus, the MHC polypeptides of this invention do not contain materials normally associated with their *in situ* environment, *e.g.*, other surface porteins on antigen presenting cells. Even where a protein has been isolated to a homogenous or dominant band, there are trace contaminants in the range of 5-10% of native protein which co-purify with the desired protein. Isolated polypeptides of this invention do not contain such endogenous co-purified protein.

The term "residue" refers to an amino acid or amino acid mimetic incorporated in a oligopeptide by an amide bond or amide bond mimetic.

### BRIEF DESCRIPTION OF THE DRAWINGS

Fig.1 provides a list of the DQ/DR haplotypes in humans and their associations with autoimmune diseases.
Fig. 2 shows the location of two peptides I-A^{s}β p18mer and I-A^{s}βp10mer and their location in the third hypervariable region of the β chain of I-A^{s}.
Fig. 3A shows the results of ELISA binding assays of antibodies obtained from animals immunized with the 18mer peptide.

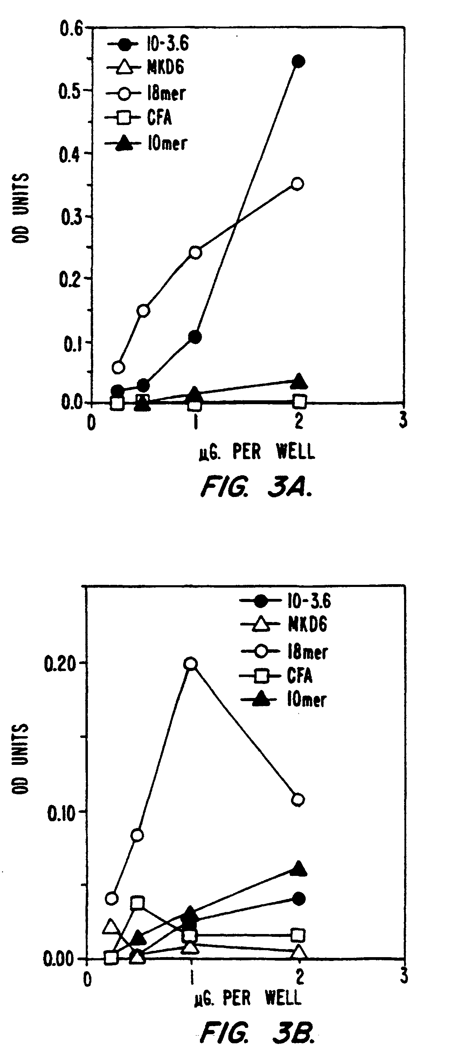

Fig. 3A shows the results of ELISA binding assays of antibodies obtained from animals immunized with the 10mer peptide.

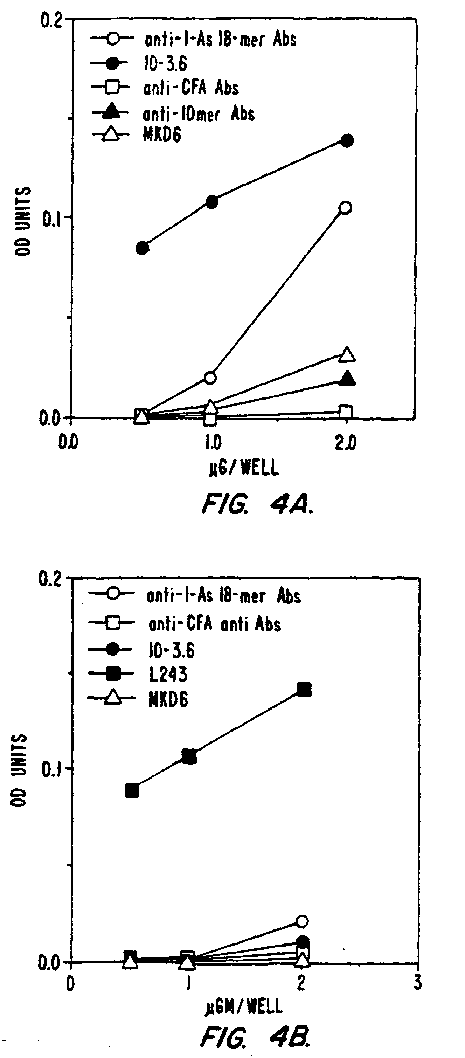

Fig. 4A shows the results of ELISA binding assays of antibodies to soluble I-A^{s}.
Fig. 4B shows the results of ELISA binding assays of antibodies to soluble DR.

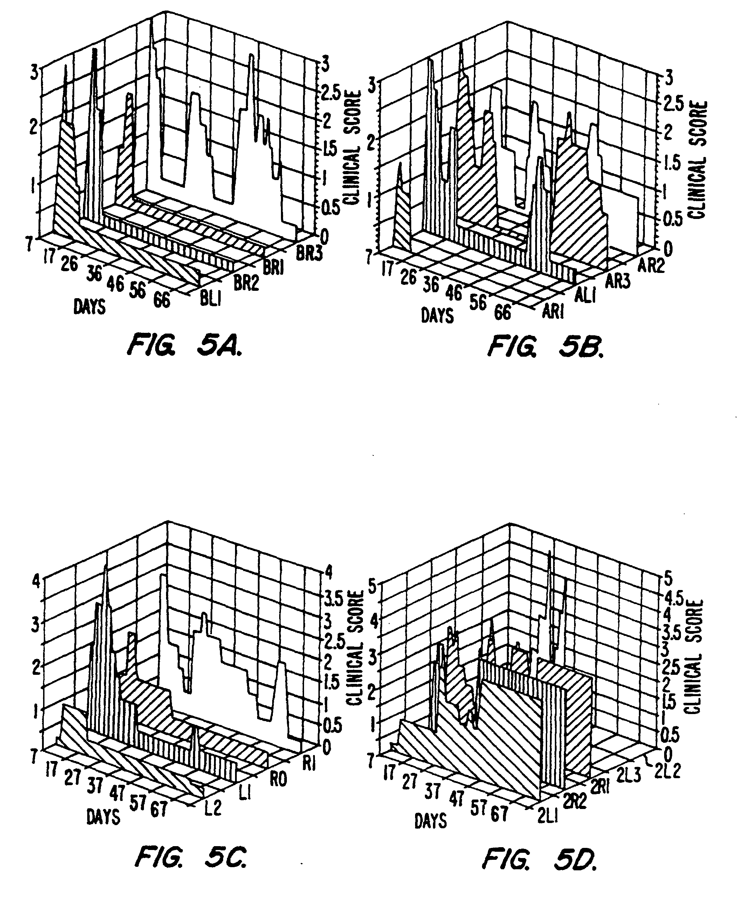

Figs. 5A and 5C shows the clinical course of CR-EAE in SJL/J mice that received the 18mer peptide in CFA.
Fig. 5B and 5D shows the clinical course of CR-EAE in SJL/J mice that received CFA alone.

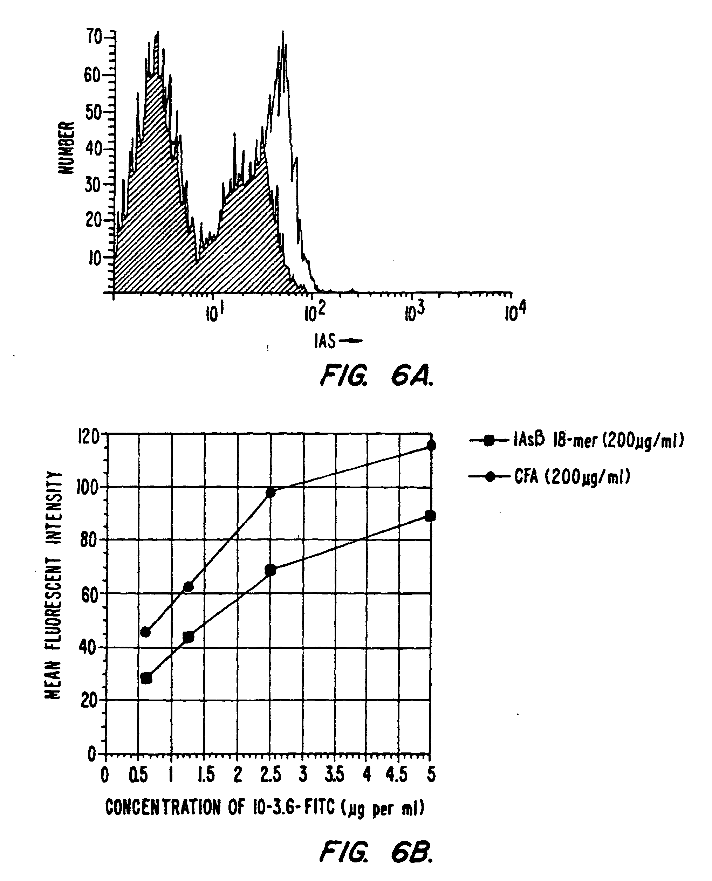

Fig. 6 shows blocking of binding of the anti-I-A^{s} monoclonal antibody 10-3.6 by anti-I-A^{s}β 18-mer peptide antiserum. This figure is a plot of mean fluorescent intensity at various concentrations of 10-3.6-FITC.

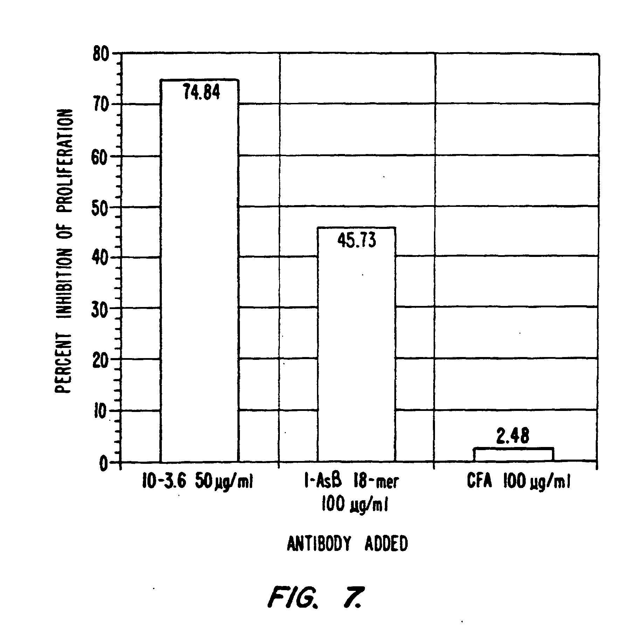

Fig. 7 shows percent inhibition of the proliferation of SJL lymph node cells to MBP p91-103 peptide by either mAb 10-3.6, anti-I-A^{s}β 18-mer peptide antiserum, or CFA control antiserum.

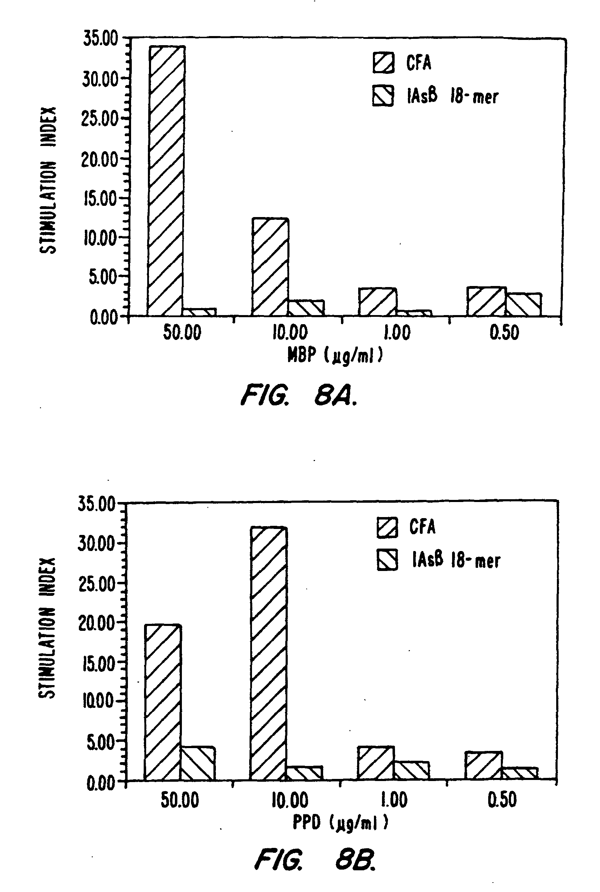

Figs. 8A and 8B show proliferative responses of regional lymph node cells to MBP (Fig. 8A) and PPD (Fig. 8B) in SJL mice that were initially vaccinated with 400µg of I-A^{s}β 18-mer in CFA, or CFA alone, and were then immunized with 400µg/animal of MBP in CFA four weeks later. Results are expressed as the stimulation index: mean cpm in wells with antigen divided by the mean cpm in wells without antigen. The mean background cpm in wells without antigen in the group that received I-A^{s}β 18-mer was 374 cpm and those that received CFA alone was 399 cpm.

### DESCRIPTION OF THE PREFERRED EMBODIMENTS

The present invention provides immunogenic polypeptides derived from the Major Histocompatibility Complex (MHC) glycoprotein protein sequences for use in compositions and methods for the treatment, prevention and diagnosis of deleterious immune responses. The polypeptides are capable of inducing an immune response against glycoproteins encoded by MHC alleles associated with the target disease. In preferred embodiments the polypeptides of the invention are derived from hypervariable regions of the α or β chain of an MHC Class II molecule associated with the deleterious immune response. In this way, the ability of antigen presenting cells (APC) to present the target antigen (*e.g.*, autoantigen or allergen) is inhibited.

The glycoproteins encoded by the MHC have been extensively studied in both the human and murine systems. Many of the histocompatibility proteins have been isolated and characterized. For a general review of MHC glycoprotein structure and function, see *Fundamental Immunology,* 3d Ed., W.E. Paul, ed., (Ravens Press N.Y. 1993).

MHC molecules are heterodimeric glycoproteins expressed on cells of higher vertebrates and play a role in immune responses. In humans, these molecules are referred to as human leukocyte antigens (HLA). MHC glycoproteins are divided into two groups, class I and class II, which differ structurally and functionally from each other. In general, the major function of MHC molecules is to bind antigenic peptides and display them on the surface of cells.

Class I MHC molecules are expressed on almost all nucleated cells and are recognized by cytotoxic T lymphocytes, which then destroy the antigen-bearing cells. Class II MHC molecules are expressed primarily on cells involved in initiating and sustaining immune responses, such as T lymphocytes, B lymphocytes, macrophages, and the like. Class II MHC molecules are recognized by helper T lymphocytes and induce proliferation of helper T lymphocytes and amplification of the immune response to the particular antigenic peptide that is displayed.

Engagement of the T cell receptor induces a series of molecular events characteristic of cell activation, such as, increase in tyrosine phosphorylation, Ca ⁺⁺ influx, PI turnover, synthesis of cytokines and cytokine receptors, and cell division (see, Altman et al., (1990) *Adv. Immunol.* 48:227-360. For a general discussion of how T cells recognize antigen see Grey, H.M., et al., *Scientific American* pp 56-64, (November, 1989).

In mice, Class I molecules are encoded by the K, D and Qa regions of the MHC. Class II molecules are encoded by the I-A and I-E subregions. The isolated antigens encoded by the murine I-A and I-E subregions have been shown to consist of two noncovalently bonded peptide chains: an α chain of 32-38 kd and a β chain of 26-29 kd. A third, invariant, 31 kd peptide is noncovalently associated with these two peptides, but it is not polymorphic and does not appear to be a component of the antigens on the cell surface. The α and β chains of a number of allelic variants of the I-A region have been cloned and sequenced.

The human Class I proteins have also been studied. The MHC Class I of humans on chromosome 6 has three loci, HLA-, HLA-B, and HLA-C, the first two of which have a large number of alleles encoding alloantigens. These are found to consist of a 44 kd subunit and a 12 kd β₂-microglobulin subunit which is common to all antigenic specificities. Further work has resulted in a detailed picture of the 3-D structure of HLA-A2, a Class I human antigen. (Bjorkman, P.J., et al., (1987) *Nature* 329:506-512). In this picture, the β₂-microglobulin protein and α₃ domain of the heavy chain are associated. The α₁ and α₂ domains of the heavy chain comprise the hypervariable region which forms the antigen-binding sites to which the peptide is bound.

Human Class II (encoded by alleles at the HLA-DR, -DP, and DQ loci) glycoproteins have a domain structure, including antigen binding sites, similar to that of Class I. The Class II molecules comprise two chains, the α and β chains, which extend from the membrane bilayer. As with the Class I molecules, each subunit in Class II molecules consist of globular domains, referred to as α1, α2, β1, and β2. All except the α1 domain are stabilized by intrachain disulfide bonds typical of molecules in the immunoglobulin superfamily. The N-terminal portions of the α and β chains, the α1 and β1 domains, contain hypervariable regions which are thought to comprise the majority of the antigen-binding sites (*see,* Brown *et al., Nature* 364:33-39 (1993)).

As noted above, each MHC allele encodes proteins which comprise hypervariable regions and antigen binding sites specific for particular sets of antigenic peptides. If the peptides bound by the MHC molecule are from an autoantigen, allergen or other protein associated with a deleterious immune response, the hypervariable region of the MHC molecule can be used to produce immunogenic polypeptides which will elicit an immune response against the MHC molecule. These polypeptides are therefore useful in targeting particular gene products associated with deleterious immune responses because the immune response against the MHC molecule will inhibit antigen presentation associated with the deleterious immune response.

Thus, immunization with the polypeptides will be haplotype specific and result only in the inhibition of the immune response mediated by the target molecules, while leaving other alleles unaffected. Most individuals are heterozygous at each MHC locus, *e.g*., the HLA-DR locus. Therefore, haplotype specific therapy of disease by immunization with polypeptides of the disease susceptibility gene products of MHC genes offers a novel means of immunotherapy. This therapy is unlikely to bring about global immunosuppression since other alleles at the particular locus will be unaffected.

Polypeptides suitable for use in the present invention can be obtained in a variety of ways. Conveniently, they can be synthesized by conventional techniques employing automatic synthesizers, such as the Beckman, Applied Biosystems, or other commonly available peptide synthesizers using well known protocols. They can also be synthesized manually using techniques well known in the art. See, e.g. Stewart and Young, *Solid Phase Peptide Synthesis,* (Rockford, Ill., Pierce), 2d Ed. (1984), which is incorporated herein by reference.

Alternatively, DNA sequences which encode the particular MHC polypeptide may be cloned and expressed to provide the peptide. Cells comprising a variety of MHC genes are readily available, for instance, they may be obtained from the American Type Culture Collection ("Catalogue of Cell Lines and Hybridomas," 6th edition (1988) Rockville, Maryland, U.S.A. Standard techniques can be used to screen cDNA libraries to identify sequences encoding the desired sequences (see, Sambrook et al., *Molecular Cloning - A Laboratory Manual,* Cold Spring Harbor Laboratory, Cold Spring Harbor, New York, 1989, which is incorporated herein by reference). Fusion proteins (those consisting of all or part of the amino acid sequences of two or more proteins) can be recombinantly produced. In addition, using *in vitro* mutagenesis techniques, unrelated proteins can be mutated to comprise the appropriate sequences.

MHC glycoproteins from a variety of natural sources are also conveniently isolated using standard protein purification techniques. Peptides can be purified by any of a variety of known techniques, including, for example, reverse phase high-performance liquid chromatography (HPLC), ion-exchange or immunoaffinity chromatography, separation be size, or electrophoresis (See, generally, Scopes, R., *Protein Purification,* Springer-Verlag, N.Y. (1982), which is incorporated herein by reference).

It will be understood that the immunogenic MHC polypeptides of the present invention may be modified to provide a variety of desired attributes, e.g., improved pharmacological characteristics, while increasing or at least retaining substantially all of the biological activity of the unmodified peptide. For instance, the peptides can be modified by extending, decreasing the amino acid sequence of the peptide. Substitutions with different amino acids or amino acid mimetics can also be made.

The individual residues of the immunogenic MHC polypeptides can be incorporated in the peptide by a peptide bond or peptide bond mimetic. A peptide bond mimetic of the invention includes peptide backbone modifications well known to those skilled in the art. Such modifications include modifications of the amide nitrogen, the α-carbon, amide carbonyl, complete replacement of the amide bond, extensions, deletions or backbone crosslinks. See, generally, Spatola, *Chemistry and Biochemistry of Amino Acids, Peptides and Proteins,* Vol. VII (Weinstein ed., 1983). Several peptide backbone modifications are known, these include, ψ[CH₂S], ψ[CH₂NH], ψ[CSNH₂], ψ[NHCO], ψ[COCH₂] and ψ[(E) or (Z) CH=CH]. The nomenclature used above, follows that suggested by Spatola, above. In this context, ψ indicates the absence of an amide bond. The structure that replaces the amide group is specified within the brackets.

Amino acid mimetics may also be incorporated in the peptides. An "amino acid mimetic" as used here is a moiety other than a naturally occurring amino acid that conformationally and functionally serves as a substitute for an amino acid in a polypeptide of the present invention. Such a moiety serves as a substitute for an amino acid residue if it does not interfere with the ability of the peptide to illicit an immune response against the appropriate MHC molecule. Amino acid mimetics may include non-protein amino acids, such as β-γ-δ-amino acids, β-γ-δ-imino acids (such as piperidine-4-carboxylic acid) as well as many derivatives of L-α-amino acids. A number of suitable amino acid mimetics are known to the skilled artisan, they include cyclohexylalanine, 3-cyclohexylpropionic acid, L-adamantyl alanine, adamantylacetic acid and the like. Peptide mimetics suitable for peptides of the present invention are discussed by Morgan and Gainor, (1989) *Ann. Repts. Med. Chem.* 24:243-252/

As noted above, the peptides employed in the subject invention need not be identical, but may be substantially identical, to the corresponding sequence of the target MHC molecule. Therefore, the peptides may be subject to various changes, such as insertions, deletions, and substitutions, either conservative or non-conservative, where such changes might provide for certain advantages in their use. The polypeptides of the invention can be modified in a number of ways so long as they comprise a sequence substantially identical (as defined below) to a sequence in the target region of the the MHC molecule.

Alignment and comparison of relatively short amino acid sequences (less than about 30 residues) is typically straightforward. Comparison of longer sequences may require more sophisticated methods to achieve optimal alignment of two sequences. Optimal alignment of sequences for aligning a comparison window may be conducted by the local homology algorithm of Smith and Waterman (1981) *Adv. Appl. Math.* 2:482, by the homology alignment algorithm of Needleman and Wunsch (1970) *J. Mol. Biol.* 48:443, by the search for similarity method of Pearson and Lipman (1988) *Proc. Natl. Acad. Sci. (USA)* 85:2444, by computerized implementations of these algorithms (GAP, BESTFIT, PASTA, and TFASTA in the Wisconsin Genetics Software Package Release 7.0, Genetics Computer Group, 575 Science Dr., Madison, WI), or by inspection, and the best alignment (*i.e.*, resulting in the highest percentage of sequence similarity over the comparison window) generated by the various methods is selected.

The term "sequence identity" means that two polynucleotide sequences are identical (*i.e.*, on a nucleotide-by-nucleotide basis) over a window of comparison. The term "percentage of sequence identity" is calculated by comparing two optimally aligned sequences over the window of comparison, determining the number of positions at which the identical residues occurs in both sequences to yield the number of matched positions, dividing the number of matched positions by the total number of positions in the window of comparison (*i.e*., the window size), and multiplying the result by 100 to yield the percentage of sequence identity.

As applied to polypeptides, the term "substantial identity" means that two peptide sequences, when optimally aligned, such as by the programs GAP or BESTFIT using default gap weights, share at least 80 percent sequence identity, preferably at least 90 percent sequence identity, more preferably at least 95 percent sequence identity or more (*e.g.*, 99 percent sequence identity). Preferably, residue positions which are not identical differ by conservative amino acid substitutions. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are: valine-leucine-isoleucine, phenylalanine-tyrosine, lysine-arginine, alanine-valine, and asparagine-glutamine.

The polypeptides of the invention typically comprise at least 18 residues. In certain embodiments the peptides will not exceed about 50 residues and typically will not exceed about 20 residues.

Since the polypeptides of the invention are typically derived from self proteins, *i.e*., MHC molecules involved in presenting antigens associated with immune pathologies, host immune response against the polypeptides of the invention may vary. It has been shown, however, that synthetic peptides of MHC Class I molecules can induce a specific cytotoxic T cell response (Maryanski *et al., Nature* 324:578 (1986)).

It is known that self peptides are continuously processed and presented by antigen presenting cells in the context of self-MHC molecules. In most instances, responses to these proteins are restricted to a limited number of epitopes. T cell selection is the consequence of the interaction of the self MHC-peptide complexes and developing T cells in the thymus. Although deletion of T cells reactive with self proteins occurs, it is not absolute and some reactivity to self peptides remains. The mechanisms by which T cells recognizing self proteins remains is unclear. Without wishing to be bound by theory, one possible explanation is that since processing of proteins is a prerequisite for T cell activation, not all combinations of peptides are presented during normal antigen processing. Those determinants not presented to T cells are referred to here as "cryptic".

The results presented below show that polypeptides of the invention derived from self MHC molecules do induce antibodies against self MHC molecules. It is thus conceivable that these polypeptides do not have natural counterparts in antigen presenting cells *in vivo.* Thus, polypeptides derived from self MHC molecules which comprise such cryptic determinants of whole molecules are likely to remain immunogenic while the parent molecules may be tolerated by the immune system.

### Selection of MHC Molecules for Therapy

In order to select the MHC molecules for producing peptides of the invention, particular MHC molecules which are involved in the presentation of the antigen are identified.

In the case of allergies, specific allergic responses are correlated with specific MHC types. For instance, allergic reactions to ragweed are known to be associated with DR2 alleles. Marsh et al., (1989) *Cold Spring Harb Symp Quant Biol* 54:459-70, which is incorporated herein by reference.

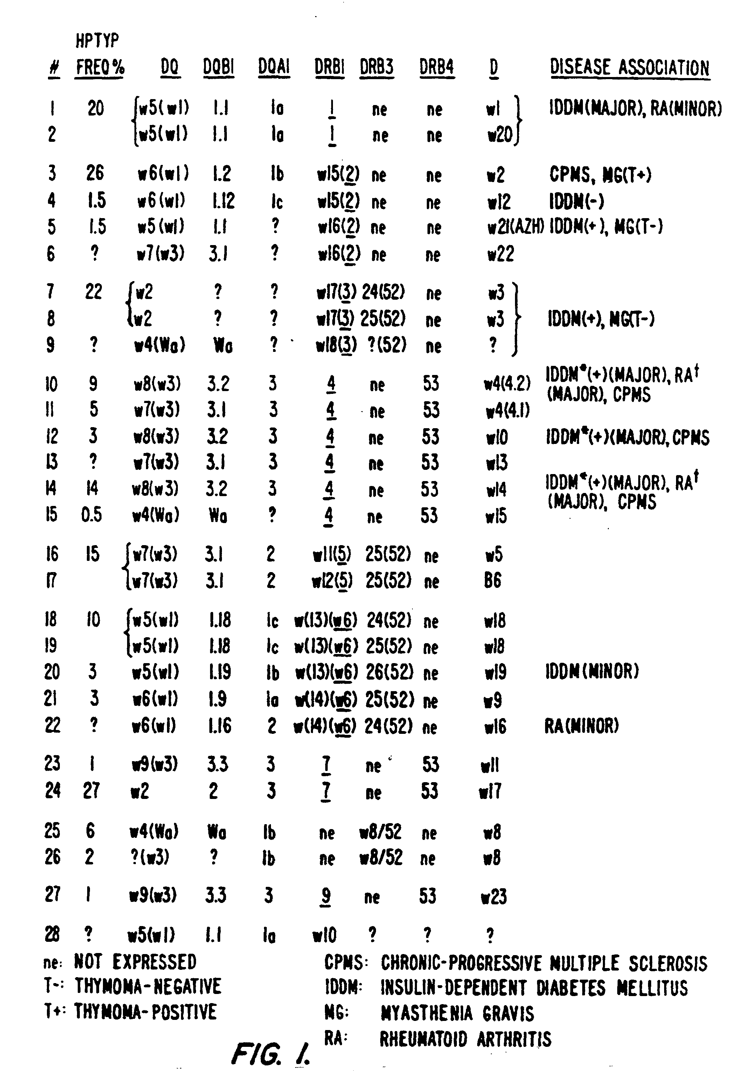

Specific autoimmune dysfunctions are also correlated with specific MHC types. A list of the DQ/DR haplotypes in humans and their associations with autoimmune diseases are shown in Figure 1. Methods for identifying which alleles, and subsequently which MHC encoded polypeptides, are associated with an autoimmune disease are known in the art. Suitable methods are described, for instance, in EP publication No. 286447. In this method several steps are followed.

First, the association between an MHC antigen and the autoimmune disease is determined based upon genetic studies. The methods for carrying out these studies are known to those skilled in the art, and information on all known HLA disease associations in humans is maintained in the HLA and Disease Registry in Copenhagen. The locus encoding the polypeptide associated with the disease is the one that would bear the strongest association with the disease.

Second, specific alleles encoding the disease associated with MHC antigen are identified. In the identification of the alleles, it is assumed that the susceptibility allele is dominant. Identification of the allele is accomplished by determining the strong positive association of a specific subtype with the disease. This may be accomplished in a number of ways, all of which are known to those skilled in the art. E.g., subtyping may be accomplished by mixed lymphocyte response (MLR) typing and by primed lymphocyte testing (PLT). Both methods are described in Weir and Blackwell, eds., Handbook of Experimental Immunology. It may also be accomplished by analyzing DNA restriction fragment length polymorphism (RFLP) using DNA probes that are specific for the MHC locus being examined. Methods for preparing probes for the MHC loci are known to those skilled in the art. See, e.g., Gregersen et al. (1986), *Proc. Natl. Acad. Sci. USA* 79:5966, which is incorporated herein by reference.

The most complete identification of subtypes conferring disease susceptibility is accomplished by sequencing of genomic DNA of the locus, or cDNA to mRNA encoded within the locus. The DNA which is sequenced includes the section encoding the hypervariable regions of the MHC encoded polypeptide. Techniques for identifying specifically desired DNA with a probe, for amplification of the desired region are known in the art, and include, for example, the polymerase chain reaction (PCR) technique.

As an example, over 90% of rheumatoid arthritis patients have a haplotype of DR4(Dw4), DR4(Dw14) or DR1 (See Figure 1).

### Model Systems for In vivo Testing

The following are model systems for autoimmune diseases which can be used to evaluate the effects of the immunogenic peptides of the invention on these conditions.

### Systemic Lupus Erythematosus (SLE)

F₁ hybrids of autoimmune New Zealand black (NZB) mice and the phenotypically normal New Zealand White (NZW) mouse strain develop severe systemic autoimmune disease, more fulminant than that found in the parental NZB strain. These mice manifest several immune abnormalities, including antibodies to nuclear antigens and subsequent development of a fatal, immune complex-mediated glomerulonephritis with female predominance, remarkably similar to SLE in humans. Knight, et al., (1978) *J. Exp. Med.* 147:1653, which is incorporated hereby by reference.

In both the human and murine forms of the disease, a strong association with MHC gene products has been reported. HLA-DR2 and HLA-DR3 individuals are at a higher risk than the general population to develop SLE (Reinertsen, et al., (1970) *N. Engl. J. Med* 299:515), while in NZB/W F₁ mice (H-2^{d/u}), a gene linked to the h-2^{u} haplotype derived from the NZW parent contributes to the development of the lupus-like nephritis.

The effect of the immunogenic peptides of the invention can be measured by survival rates and by the progress of development of the symptoms, such as proteinuria and appearance of anti-DNA antibodies.

### Myasthenia Gravis (MG)

Myasthenia gravis is one of several human autoimmune diseases linked to HLA-D. McDevitt, et al., *Arth. Rheum.* (1977) 20:59 which is incorporated herein by reference. In MG, antibodies to the acetyl choline receptors (AcChoR) impair neuromuscular transmission by mediating loss of AcChoR in the postsynaptic membrane.

SJL/J female mice are a model system for human MG. In these animals, experimental autoimmune myasthenia gravis (EAMG) is induced by immunizing the mice with soluble AcChoR protein from another species. Susceptibility to EAMG is linked in part to the MHC and has been mapped to the region within H-2. Christadoss, et al., (1979) *J. Immunol.* 123:2540.

AcChoR protein is purified from *Torpedo californica* and assayed according to the method of Waldor, et al., (1983) *Proc. Natl. Acad. Sci.* 80:2713, incorporated by reference. Emulsified AcChoR, 15 ug in complete Freund adjuvant, is injected intradermally among six sites on the back, the hind foot pads, and the base of the tail. Animals are re-immunized with this same regimen 4 weeks later.

Evaluation can be made by measurement of anti-AcChoR antibodies, Anti-AcChoR antibody levels are measured by a microliter ELISA assay as described in Waldor, et al., above. The standard reagent volume is 50 ul per well. Reagents are usually incubated in the wells for 2 hr at RT. Five ug of AcChoR diluted in bicarbonate buffer, pH 9.6, is added to each well. After incubation with AcChoR, the plates are rinsed four times with a wash solution consisting of phosphate-buffer saline containing 0.05% Tween and 0.05% NaN₃. Mouse sera are diluted in 0.01M PBS (pH 7.2), 1.5 mfr MgCl₂, 2.0 mM 2-mercaptoethanol, .05% Tween-80, .05% NaN₃ (P-Tween buffer) and incubated on the plate. After the plate is washed, β-galactosidase-conjugated sheep anti-mouse antibody diluted in P-Tween buffer is added to each well. After a final washing, the enzyme substrate, p-nitrophenyl-galctopyranoside is added to the plate, and the degree of substrate catalysis is determined from the absorbance at 405 nm after 1 hr.

Anti-AcChoR antibodies are expected to be present in the immunized with AcChoR mice as compared to nonimmunized mice. Treatment with immunogenic peptides is expected to significantly reduce the titer of anti-AcChoR antibodies in the immunized mice.

The effect of treatment with the immunogenic peptides on clinical EAMG can also be assessed. Myasthenia symptoms include a characteristic hunched posture with drooping of the head and neck, exaggerated arching of the back, splayed limbs, abnormal walking, and difficulty in righting. Mild symptoms are present after a standard stress test, and should be ameliorated by administration of immunogenic peptides after a period of time after which antibody titer has fallen.

### Rheumatoid Arthritis (RA)

In humans, susceptibility to rheumatoid arthritis is associated with HLA D/DR. The immune response in mice to native type II collagen has been used to establish an experimental model for arthritis with a number of histological and pathological features resembling human RA. Susceptibility to collagen-induced arthritis (CIA) in mice has been mapped to the H-2 I region, particularly the I-A subregion. Huse, et al., (1984) *Fed*. *Proc.* 43:1820.

Mice from a susceptible strain, DBA-1 are caused to have CIA by treatment of the mice with native type II collagen, using the technique described in Wooley and Luthra, (1985) *J. Immunol.* 134:2366, incorporated herein by reference.

In another model, adjuvant arthritis in rats is an experimental model for human arthritis, and a prototype of autoimmune arthritis triggered by bacterial antigens, Holoschitz, et al., *Prospects of Immunology* (CRC Press) (1986); Pearson *Arthritis Rheum.* (1964) 7:80. The disease the result of a cell-mediated immune response, as evidenced by its transmissibility by a clone of T cells which were reactive against the adjuvant (MT); the target self-antigen in the disease, based upon studies with the same cloned cells, appears to be part(s) of a proteoglycan molecule of cartilage.

Adjuvant disease in rats is produced as described by Pearson, i.e., by a single injection of Freund's adjuvant (killed tubercle bacilli or chemical fractions of it, mineral oil, and an emulsifying agent) given into several depot sites, preferably intracutaneously or into a paw or the base of the tail. The adjuvant is given in the absence of other antigens.

The effect of immunogenic peptide treatment of manifestations of the disease are monitored. These manifestations are histopathological, and include an acute and subacute synovitis with proliferation of synovial lining cells, predominantly a mononuclear infiltration of the articular and particular tissues, the invasion of bone and articular cartilage by connective tissue pannus, and periosteal new bone formation, especially adjacent to affected joints. In severe or chronic cases, destructive changes occur, as do fibrous or bony ankylosis. These histopathological symptoms are expected to appear in control animals at about 12 days after sensitization to the Freund's adjuvant.

### Insulin Dependent Diabetes Mellitus (IDDM)

IDDM is observed as a consequence of the selective destruction of insulin-secreting cells within the Islets of Langerhans of the pancreas. Involvement of the immune system in this disease is suggested by morphologic evidence of early infiltration of the Islets by mononuclear cells, by the detection of anti-islet cell antibodies, by the high frequency of HLA-DR3 and -DR4 alleles in IDDM populations, and by clinical associations between IDDM and various autoimmune diseases. An animal model for spontaneous IDDM and thyroiditis has been developed in the BB rat. As in humans, the rat disease is controlled in part by the genes encoding the MHC antigens, is characterized by islet infiltration, and is associated with the presence of anti-islet antibodies. The I-E equivalent Class II MHC antigens appear to be involved in manifestation of the autoimmune diseases in the BB rat. Biotard, et al., *Proc. Natl. Acad. Sci. USA* (1985) 82:6627.

In morphologic evaluation, insulitis is characterized by the presence of mononuclear inflammatory cells within the islets. Thyroiditis is characterized by focal interstitial lymphocytic infiltrate within the thyroid gland, as a minimum criterion. Most severe cases show diffuse extensive lymphocytic infiltrates, disruption of acini, fibrosis, and focal Hurthle call change. See Biotard et al.

Treatment of the BB rats with immunogenic peptides of the invention is expected to ameliorate or prevent the manifestation of the clinical and morphological symptoms associated with IDDM and thyroiditis.

In another spontaneous model, the NOD mouse strain (H-2K^{d}D^{b}) is a murine model for autoimmune IDDM. The disease in these animals is characterized by anti-islet cell antibodies, severe insulitis, and evidence for autoimmune destruction of the β-cells. Kanazawa, et al., *Diabetologia* (1984) 27:113. The disease can be passively transferred with lymphocytes and prevented by treatment with cyclosporin-A (Ikehara, et al., *Proc. Natl. Acad. Sci. USA* (1985) 82:7743; Mori, et al.), *Diabetologia* (1986) 29:244. Untreated animals develop profound glucose intolerance and ketosis and succumb within weeks of the onset of the disease. Seventy to ninety percent of female and 20-30% of male animals develop diabetes within the first six months of life. Breeding studies have defined at least two genetic loci responsible for disease susceptibility, one of which maps to the MHC. Characterization of NOD Class II antigens at both the serologic and molecular level suggest that the susceptibility to autoimmune disease is linked to I-Aβ. Acha-Orbea and McDevitt, *Proc. Natl. Acad. Sci. USA* (1970) 84:235.

Treatment of Female NOD mice with immunogenic peptides is expected to lengthen the time before the onset of diabetes and/or to ameliorate or prevent the disease.

### Experimental Allergic Encephalomyelitis (EAE)

Experimental allergic encephalomyelitis (EAE) is an induced autoimmune disease of the central nervous system which mimics in many respects the human disease of multiple sclerosis (MS). The disease can be induced in many species, including mice and rats.

The disease is characterized by the acute onset of paralysis. Perivascular infiltration by mononuclear cells in the CNS is observed in both mice and rats. Methods of inducing the disease, as well as symptomology, are reviewed in Aranson (1985) in *The Autoimmune Diseases* (eds. Rose and Mackay, Academic Press, Inc.) pp. 399-427, and in Acha-Orbea et al. (1989), *Ann. ReV. Imm.* 7:377-405.

One of the genes mediating susceptibility is localized in the MHC class II region (Moore et al. (1980), *J. Immunol.* 124:1815-1820). The best analyzed encephalitogenic protein is myelin basic protein (MBP), but other encephalitogenic antigens are found in the brain. The immunogenic epitopes have been mapped (see Acha-Orbea et al., supra.). In the PL mouse strains (H-2u) two encephalitogenic peptides in MBP have been characterized: MBP peptide p35-47 (MBP 35-47), and acetylated (MBP 1-9). In humans, preferred autoantigenic peptides for treatment of MS comprise amino aids 84-102 and 148-162 of MBP.

The effect of the immunogenic peptides of the invention on ameliorating and preventing disease symptoms in individuals in which EAE has been induced can be measured by survival rates, and by the progress of the development of symptoms. An example of the use of immunogenic peptides in the treatment of EAE is provided below.

### Formulation and Administration

The peptides of the present invention and pharmaceutical compositions thereof are useful for administration to mammals, particularly humans, to treat and/or prevent deleterious immune responses. Suitable formulations are found in Remington's Pharmaceutical Sciences, Mack Publishing Company, Philadelphia, PA, 17th ed. (1985), which is incorporated herein by reference.

The immunogenic peptides of the invention are administered prophylactically or to an individual already suffering from the disease. The compositions are administered to a patient in an amount sufficient to elicit an effective immune response to the MHC molecule from which the peptides are derived. An amount adequate to accomplish this is defined as "therapeutically effective dose" or "immunogenically effective dose." Amounts effective for this use will depend on, e.g., the peptide composition, the manner of administration, the stage and severity of the disease being treated, the weight and general state of health of the patient, and the judgment of the prescribing physician, but generally range for the initial immunization (that is for therapeutic or prophylactic administration) from about 0.1 mg to about 1.0 mg per 70 kilogram patient, more commonly from about 0.5 mg to about 0.75 mg per 70 kg of body weight. Boosting dosages are typically from about 0.1 mg to about 0.5 mg of peptide using a boosting regimen over weeks to months depending upon the patient's response and condition. A suitable protocol would include injection at time 0, 2, 6, 10 and 14 weeks, followed by booster injections at 24 and 28 weeks.

It must be kept in mind that the peptides and compositions of the present invention may generally be employed in serious disease states, that is, life-threatening or potentially life threatening situations. In such cases, in view of the minimization of extraneous substances and the relative nontoxic nature of the peptides, it is possible and may be felt desirable by the treating physician to administer substantial excesses of these peptide compositions.

For therapeutic use, administration should begin at the first sign of autoimmune or allergic disease. This is followed by boosting doses until at least symptoms are substantially abated and for a period thereafter. In some circumstances, loading doses followed by boosting doses may be required. The resulting immune response helps to cure or at least partially arrest symptoms and/or complications. Vaccine compositions containing the peptides are administered prophylactically to a patient susceptible to or otherwise at risk of the disease to elicit an immune response against the target MHC antigen.

The pharmaceutical compositions are intended for parenteral or oral administration. Preferably, the pharmaceutical compositions are administered parenterally, e.g., subcutaneously, intradermally, or intramuscularly. Thus, the invention provides compositions for parenteral administration which comprise a solution of the immunogenic peptides dissolved or suspended in an acceptable carrier, preferably an aqueous carrier. A variety of aqueous carriers may be used, e.g., water, buffered water, 0.4% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as buffering agents, tonicity adjusting agents, wetting agents and the like, for example, sodium acetate, sodium lactate, sodium chloride, potassium chloride, calcium chloride, sorbitan monolaurate, triethanolamine oleate, etc.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more peptides of the invention, and more preferably at a concentration of 25%-75%.

As noted above, the compositions are intended to induce an immune response to the peptides. Thus, compositions and methods of administration suitable for maximizing the immune response are preferred. For instance, peptides may be introduced into a host, including humans, linked to a carrier or as a homopolymer or heteropolymer of active peptide units. Alternatively, the a "cocktail" of polypeptides can be used. A mixture of more than one polypeptide has the advantage of increased immunological reaction and, where different peptides are used to make up the polymer, the additional ability to induce antibodies to a number of epitopes. For instance, polypeptides comprising sequences from hypervariable regions of α and β chains may be used in combination. Useful carriers are well known in the art, and include, e.g., thyroglobulin, albumins such as human serum albumin, tetanus toxoid, polyamino acids such as poly(lysine:glutamic acid), influenza, hepatitis B virus core protein, hepatitis B virus recombinant vaccine and the like.

The use of more than one polypeptide is particularly useful to enhance the immune response against polypeptides of the invention. As demonstrated below, although the polypeptides may be derived from self MHC molecules expressed in the patient, they can induce an immune response. In some instances, the immune response to the self polypeptide may not be sufficiently strong. In these instances, it may be necessary to break tolerance to the polypeptide. the compositions may comprise one or more of the foreign polypeptides that are sufficiently similar to the self polypeptides to induce an immune response against both the foreign and self polypeptides (*see,* Mamula *et al. J. Immunol.* 149:789-795 (1992). Suitable proteins include synthetic polypeptides designed for this purpose or polypeptide sequences from homologous proteins from natural sources, such as proteins encoded by a different allele at the same locus as the self polypeptide.

The compositions also include an adjuvant. A number of adjuvants are well known to one skilled in the art. Suitable adjuvants include incomplete Freund's adjuvant, alum, aluminum phosphate, aluminum hydroxide, N-acetyl-muramyl-L-threonyl-D-isoglutamine (thr-MDP), N-acetyl-nor-muramyl-L-alanyl-D-isoglutamine (CGP 11637, referred to as nor-MDP), N-acetylmuramyl-L-alanyl-D-isoglutaminyl-L-alanine-2- (1'-2'-dipalmitoyl-sn-glycero-3-hydroxyphosphoryloxy)-ethylamine (CGP 19835A, referred to as MTP-PE), and RIBI, which contains three components extracted from bacteria, monophosphoryl lipid A, trehalose dimycolate and cell wall skeleton (MPL+TDM+CWS) in a 2% squalene/Tween 80 emulsion. The effectiveness of an adjuvant may be determined by measuring the amount of antibodies directed against the immunogenic peptide.

A particularly useful adjuvant and immunization schedule are described in Kwak et al. *New Eng. J. Med.* 327-1209-1215 (1992). The immunological adjuvant described there comprises 5% (wt/vol) squalene, 2.5% Pluronic L121 polymer and 0.2 % polysorbate in phosphate buffered saline.

The concentration of immunogenic peptides of the invention in the pharmaceutical formulations can vary widely, i.e. from less than about 0.1%, usually at or at least about 2% to as much as 20% to 50% or more by weight, and will be selected primarily by fluid volumes, viscosities, etc., in accordance with the particular mode of administration selected.

The peptides of the invention can also be expressed by attenuated viral hosts, such as vaccinia or fowlpox. This approach involves the use of vaccinia virus as a vector to express nucleotide sequences that encode the peptides of the invention. Upon introduction into a host, the recombinant vaccinia virus expresses the immunogenic peptide, and thereby elicits an immune response. Vaccinia vectors and methods useful in immunization protocols are described in, e.g., U.S. Patent No. 4,722,848. Another vector is BCG (Bacille Calmette Guerin). BCG vectors are described in Stover et al. (*Nature* 351:456-460 (1991)). A wide variety of other vectors useful for therapeutic administration or immunization of the peptides of the invention, e.g., *Salmonella typhi* vectors and the like, will be apparent to those skilled in the art from the description herein.

The peptides can also be used for diagnostic purposes. For instance, they can be used to screen for autoantibodies to ensure that the vaccination has been effective.

The following examples are offered by way of illustration, not by way of limitation.

### Example 1

This example shows that immunization of mice with peptides of the invention elicit an immune response to the target MHC antigen.

The model system used was Experimental Autoimmune Encephalomyelitis (EAE). As explained above, EAE is an animal model of a T cell mediated autoimmune demyelinating disease that resembles human Multiple Sclerosis (MS). The disease is characterized by the development of an acute paralytic attack followed by recovery. Spontaneous remissions followed by variable recovery are seen when animals are observed over a three month period. In view of these features EAE is an ideal model for the study of immunotherapy of chronic autoimmune disease.

Like MS, susceptibility to EAE is linked to certain alleles of mouse la genes, with I-A^{s,u,&k} strains being susceptible while I-A^{b&d} strains relatively resistant. EAE can be prevented and the severity of CR-EAE reduced, following treatment with monoclonal anti-I-A antibody 10-3.6 (Sriram, et al. (1983) *J. Exp. Med*., 158:1362). Monoclonal antibody 10-3.6 recognizes the serological specificity 1a17, on the β chain of I-A molecule, binding to residues 63-67 of the β chain of the alleles of IA^{s,u,f,r} and ^{k11}.

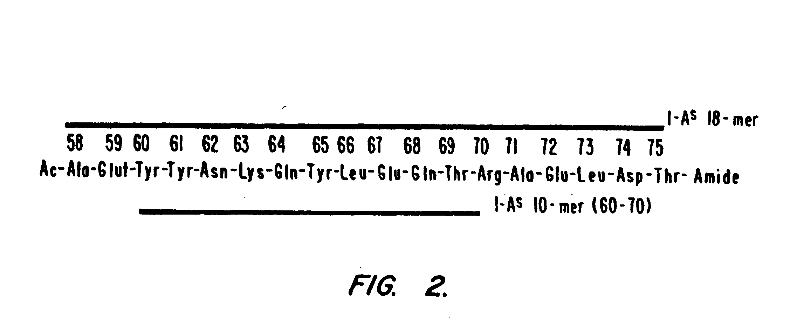

Synthetic peptides that spanned the monoclonal antibody 10-3.6 binding site on the β chain of I-A^{s} were generated. These peptides were I-A^{sβ} p18mer, spanning residues 58-75 and I-A^{sβ}p10mer spanning residues 60-70 of the third hypervariable region of the β chain (Figure 2). The peptides were obtained from (Macromolecular Resources, Colorado State Univ, Fort Collins CO).

The results of ELISA binding assays of antibodies obtained from animals immunized with the 18mer and the 10mer are shown in Figures 3A and 3B, respectively. Five female SJL mice, 8 weeks of age (obtained from NIH, Bethesda, MD) were immunized on the dorsum with 350µgm of the peptide in complete Freund's Adjuvant containing 50µg of H37RA (CFA). The animals were re-immunized with 200µg of the peptide 7 days later and bled via tail vein 3 weeks after the second immunization. Control animals were immunized with CFA alone or with an irrelevant 20mer peptide (pb 57, a 20mer peptide of thrombin, gift of W. Church, University of Vermont, Burlington VT). The sera were pooled from five animals and the immunoglobulins were precipitated with supersaturated ammonium sulphate according to standard procedures. Solubilized precipitate was further purified by chromatography over a QAE column and quantified by absorbance reading 280nm on a spectrophotometer.

ELISA assays were performed by coating ELISA plates (Corning, NY) with antigen (2µg/ well 10-mer peptide or with 1µgm/well of the 18-mer peptide) in 100µl of bicarbonate buffer (pH9.2) overnight. The wells were washed in ELISA washing buffer (PBS with 0.05% Tween 20), unoccupied sites blocked with 1% bovine serum albumin (Sigma, St. Louis, M0) in PBS for 30 minutes and washed. 2µg,1µg,0.5µg and 0.25µg of antibody diluted in ELISA buffer was added to each well. After 45 minutes the wells were washed and alkaline phosphatase-conjugated goat-anti-mouse lgG (Tago, Millbrae, CA) was added at a dilution of 1:5000. After 30 minutes the wells were washed and 100µl of the substrate (5mgs of p-nitrophenyl phosphate dissolved in 10% diethanolamine (Sigma) to a final concentration of 1mg/m) was added to the wells. The color reaction was read in a Bio-Tek ELIZA reader (Winooski, VT) at 405nm at 120 minutes. Results are expressed as mean absorbance of triplicate wells read at 405nm. after subtraction of background absorbance at 405nm units (Absorbance 405nm in wells to which no primary antibody was added).

Antibodies to the 18mer antigen were detected in SJL mice following immunization with the I-A^{s}β p18mer peptide (Figure 3A). The 10mer peptide was poorly immunogenic and did not result in the development of a significant antibody titre (Figure 3B). Also, monoclonal antibody 10-3.6 bound to the 18mer peptide as expected, while the control isotype-matched antibody MKD6 (which recognizes a polymorphic region of I-A^{d}) showed no binding. Only the anti 18mer antisera bound to the 10mer peptide suggesting that the anti-18mer antibody recognized a region distinct from that recognized by antibody 10-3.6. Neither peptide gave rise to a proliferative T cell response. Immunization with an irrelevant 20mer peptide (pb 57, a synthetic peptide of thrombin protein) did not elicit antibodies to either the 20mer or the 10mer peptide (data not shown).

To determine if the serum antibody was specific to IA molecules, an ELISA assay using soluble I-A molecules as the ligand was used.

Soluble 1-A^{s} protein was prepared as previously described in Sharma et al. (1991) *Proc. Natl. Acad. Sci. USA* 88:11465. Soluble DR was prepared from homozygous typing cell line GMO-3107, that is homozygous for HLA-DR2. Briefly, the DR2 typing cell line was grown in 8 liter culture flasks and at cell density of 1x10⁶ cells/ml, the cells were then harvested and a detergent lysate of the membrane preparation was passed over a column containing anti-DR antibody (L234) coupled to sepharose 4B. The bound DR molecules were eluted at pH 11.3 and the protein peaks pooled. A 12% SDS-PAGE gel was run to establish the purity of the preparation. The soluble 1-A^{s} and DR proteins were diluted in bicarbonate buffer pH9.2. 1µg of the protein in 100µl of buffer was added to the well and the ELISA assay was performed as described above.

As shown in Figure 4A, antibodies from I-A^{s}β p18mer peptide immunized animals bound to the soluble I-A^{s} antigen. Antibodies obtained from animals that were immunized with the I-Aβ p10 mer or with CFA alone showed no binding to the soluble I-A^{s}. When soluble HLA-DR2 was used (Figure 4B) as a control antigen, there was no binding of the anti I-Asβ 18mer or the 10-3.6 antibodies, but there was binding of anti HLA-DR antibody L243. These studies establish, that anti I-A specific antibodies can be generated in animals autologous for the I-A gene products, following immunization with I-A peptides.

### Example 2

This example shows that the induction of anti I-A^{s} antibody response is sufficient to prevent the development of acute and CR-EAE.

Female SJL/J mice, 6-12 weeks of age were obtained from NIH (Bethesda, MD) and maintained according to standard techniques. The mice were immunized on the back with 150 µl of an emulsion comprising either Complete Freunds Adjuvant (CFA, to which 350 µg/ml of H37RA was added, CFA with 400 µgm of I-A^{s}β p18-mer, CFA with 400 µgm of I-A^{s}β p10-mer, or CFA with 400 µgm of 57pb (20mer peptide of thrombin, irrelevant peptide).

Four weeks later all animals were challenged with 800 µgm of Mouse Spinal Cord Homogenate (MSCH) in CFA. The immunization with MSCH was repeated 7 days later and disease was monitored between days 10-20. Disease was graded as follows: (1) limp tail, (2) paralysis of one limb, (3) paralysis of two limbs, (4) moribund, (5) death. Twenty days following immunization with MSCH all animals were perfused with 4% paraformaldehyde and the brain and spinal cord obtained for histological analysis. Histology was graded as follows: 4+, greater than 6 perivascular cuffs present in 6 non-overlapping fields observed at medium power; 3+, 3-6 perivascular cuffs present in nonoverlapping fields at medium power; 2+, 1-3 perivascular cuffs present in nonoverlapping fields at medium power; 1+, meningeal infiltration only. Histology of brain including cerebellum and brain stem was studied in all animals from experiment 1.

The results of these experiments (Table 1) show that immunization with I-A^{s}β p18mer peptide protects against the development of EAE. In all, only 3 out of 16 animals (23%) that were vaccinated with the peptide I-A^{s}β p18mer developed EAE. In animals that were injected with CFA alone or CFA with p57 (an irrelevant 20mer peptide) 13 of the 16 animals (81%) developed EAE. Histological evidence of the difference in severity was also confirmed. I-A^{s}βp10mer was not successful in generating anti I-A^{s} antibody and did not prevent EAE.

**Table 1.**

| Prevention Of EAE Following Immunization With I-A^{s}β Chain Peptide 58-75. | | | | | |
|---|---|---|---|---|---|
| Treatment | No Animals | No Paralyzed | Mean Severity Of Mice Paralyzed | Day Of Onset | Histology |
| Exp. 1 | | | | | |
| CFA alone | 4 | 3 | 3.0 | 12 | 3 |
| CFA +I-A^{s} p58-75 | 4 | 0 | 0 | 0 | - |
| | | | | | |

| Exp. 2 | | | | | |
|---|---|---|---|---|---|
| CFA+ 57pb | 6 | 4 | 2.4 | 13 | Not Done |
| CFA I-A^{s} p58-75 | 6 | 1 | 3.0 | 21 | Not Done |
| | | | | | |

| Exp. 3 | | | | | |
|---|---|---|---|---|---|
| CFA alone | 6 | 6 | 2.4 | 13 | Not Done |
| I-A^{s} p58-75 | 6 | 2 | 2.0 | 16 | Not Done |
| | | | | | |

| Exp. 4 | | | | | |
|---|---|---|---|---|---|
| CFA+ I-A^{s} p60-70 (10-mer) | 6 | 6 | 3.0 | 11 | |
| CFA alone | 6 | 6 | 2.6 | 12 | |
| | | | | | |
| Total | | | | | |
| I-A^{s} 58-75 | 16 | 3¹ | 2.0 | | |
| I-A^{s} p60-70 | 6 | 6 | 3.0 | | |
| All Controls | 16 | 13 | 2.5 | | |
| ¹X2 = I-Ap (18mer) VS CFA alone (p<.0001) = I-Ap (10mer) VS CFA alone p, not significant | | | | | |

In order to determine the effect of immunization with I-A^{s}β p18mer peptides on established disease, vaccination of animals with I-A^{s}β p18mer peptide, was initiated following recovery from the initial paralytic attack (Table 2).

SJL mice 6-8 weeks of age were immunized on days 0 and 7 with 400µgms MBP peptide p91-103 (Multiple Peptide System, San Diego CA.) in CFA containing 50µgm/ml of H37RA. Fourteen days later, regional draining lymph node cells were harvested and cultured in 24 well plates (Falcon) at a concentration of 6x10⁶ cells/well in 1.5mls of RPMI 1640 medium containing 10% fetal bovine serum (Hyclone Labs, Logan, UT.), 2mM L-glutamine, 5x10⁻⁵M 2-mercaptoethanol, 1% penicillin/streptomycin, and 5µgm/ml of peptide or 10µg/ml of p91-103 peptide. Following a 4 day in vitro stimulation, antigen reactive T cell blasts were harvested via ficoll-hypaque gradient centrifugation (Hypaque 1077, Sigma, St. Louis, MO), washed twice in PBS and injected into recipient mice (1.5 x 10⁷ cells/animal in 500ul PBS, i.p.).

Animals were observed for the development of EAE and upon recovery were immunized with either 400µgm of I-A^{s}β 18 mer peptide in CFA (Group 1) or CFA alone (Group 2). Recovery was defined as an improvement of 2 clinical grades or more that was present for more than 48 hrs. In experiment 1, recovery occurred in all animals by day 17 and animals were injected with the I-A^{s}β 18 mer peptide or CFA on day 18 and in the second experiment, the animals were treated with the I-A^{s}β 18 mer peptide on day 24. Animals were followed daily up to day 75.

**Table 2**

| Clinical course of CR-EAE in animals treated with I-A^{s}β 18 mer peptide after recovery from the initial paralytic attack Summary of two experiments. | | | |
|---|---|---|---|
| | No. of Mice per Group | Mean day onset of paralysis | Mean severity |
| Initial Attack | | | |
| Group 1 | | | |
| I-A^{s}β 18 mer | 8 | 8.3 | 2.2 |
| peptide treated | | | |
| Group 2 | | | |
| CFA treated | 9 | 8.9 | 2.4 |
| | | | |

| First Relapse | | | |
|---|---|---|---|
| Group 1 | 2/8 | 27 | 1.8 |
| Group 2 | 8*/9 | 32 | 3.0 |
| | | | |

| Second Relapse | | | |
|---|---|---|---|
| Group 1 | 2/8 | 57 | 2.0 |
| Group 2 | 5/7 | 50 | 2.3 |
| | | | |

| Cumulative relapses | | | |
|---|---|---|---|
| Group 1 | 4# | | |
| Group 2 | 13 | | |

| | | | |
|---|---|---|---|
| *Two animals died in the first relapse.# p<0.05, Wilcoxan rank sum test | | | |

These studies show that overall there were only four relapses in the I-A^{s}β p18mer treated group when compared to 13 in the control group. In Experiment 2, the relapses were more severe with two deaths at the first relapse and the remaining three animals displaying Grade 2 or greater paralysis, for the remainder of the study (Figure 5). Overall, the relapse rate (Number of relapses/number of animals) in animals that received I-A^{s}βp20mer was 0.27, while those in the control group overall was 1.3 (p<0.05).

This study establishes the efficacy of vaccination with I-A^{s}β peptides as a therapeutic strategy in the treatment of autoimmune disease. The clinical effect observed here closely parallels the results obtained with in vivo therapy with anti I-A antibody in the treatment of acute and CR-EAE.

### Example 3

This example presents the results of flow cytometric analysis, T cell proliferation assays to analyze the nature of the immune response induced by polypeptides of the invention.

### The auto-anti-I-A antibodies from I-A^{s}β 18-mer peptide vaccinated animals are specific for native I-A^{s} expressed on the cell surface.

Flow cytometric analysis was performed on splenic lymphocytes to determine whether or not the antiserum from I-A^{s}β 18-mer peptide vaccinated animals could recognize native I-A^{s} molecule on the cell surface. Splenic lymphocytes containing T-cells, B-cells, and monocytes were obtained from SJL/J (I-A^{s}) and BALB/c (I-A^{d}) mice. The cells were then stained *in vitro* with purified antiserum from animals vaccinated either with I-A peptide or CFA alone. A goat anti-mouse IgG Fc conjugated to fluorescein isothyocyanate (FITC) was used as secondary antibody. Monoclonal antibody 10-3.6 conjugated to FITC was used as a positive control.

The results of these experiments indicated that 36.17% of splenic lymphocytes were stained by the I-A^{s}β 18-mer antiserum at a concentration of 50 µg/ml. This is compared to 40% of cells stained with the monoclonal anti-I-A antibody 10-3.6. In contrast only 1.91% of the cells stained with 50 µg of the CFA antiserum and 1.5% of the cells stained with anti-I-A^{d} mAb MKD6. The anti-I-A^{s}β 18-mer antiserum was specific for the SJL/J spleen cells since only 3.78% of BALB/c spleen cells were recognized.

In a separate experiment, SJL spleen cells were preincubated for 1 hr. with 200µg/ml of either the anti-I-A^{s}β 18-mer peptide antiserum or CFA control antiserum. The cells were then washed and incubated for 30 min. with FITC-conjugated 10-3.6 at concentrations of 5, 2.5, 1.25, and 0.625 µg/ml. Cells incubated with the anti-I-A^{s}β 18-mer peptide antiserum demonstrated a mean 44.4 ± 11.6% reduction in the mean fluorescent intensity at all concentrations of 10-3.6 when compared to those samples preincubated with the control antiserum (Fig. 6).

These studies establish that following vaccination with the I-A^{s}β 18-mer peptide, anti-I-A^{s} specific antibodies are generated in animals autologous for the I-A gene products.

### The auto anti-I-A antibody can inhibit Class II-restricted T-cell proliferative responses.

To determine whether the anti-I-A antibodies elicited by vaccination with I-A peptide can inhibit functional responses, a T-cell proliferative assay was performed. SJL/J mice were immunized with MBP p91-103 peptide in CFA. Nine days later the lymph nodes were removed and cultured in vitro in the presence of the p91-103 peptide. Purified antiserum from the I-A^{s}β 18-mer peptide vaccinated mice was included in the assay (100µg/ml). Alternatively, as positive and negative controls, mAb 10-3.6 (50µg/ml) and CFA antiserum (100µg/ml) were included in separate sets of wells respectively. Only the anti-I-A^{s}β 18-mer antiserum and the 10-3.6 mAb were able to inhibit proliferation (43% vs. 72% inhibition). CFA antiserum had little effect (2.48%). (Fig. 7)

### Animals vaccinated with I-A^{s}β 18-mer peptide fail to develop a proliferative response to MBP and PPD.

In order to determine if an antibody response to I-A^{s}β 18-mer peptide affects the development of immunity to soluble recall antigens, SJL mice were vaccinated with either I-A^{s}β 18-mer peptide in CFA, or CFA alone. 4 weeks later both groups receiver 400µg of MBP in CFA. Ten days after receiving MBP, the regional lymph nodes were harvested and the proliferative responses to MBP and PPD (purified protein derivative of tuberculin) were determined. Mice that had received I-A^{s}β 18-mer peptide had a significantly lowered proliferative response to both MBP and PPD when compared to the control group that received CFA alone (Fig. 8).

The above examples are provided to illustrate the invention but not to limit its scope. Other variants of the invention will be readily apparent to one of ordinary skill in the art and are encompassed by the appended claims. All publications, patents, and patent applications cited herein are hereby incorporated by reference.

## Claims

1. A composition comprising an isolated immunogenic MHC polypeptide wherein the immunogenic MHC polypeptide has a sequence from a hypervariable region of an MHC molecule associated with susceptibility to an autoimmune disease, wherein the hypervariable region is in an MHC class II β chain and wherein the immunogenic MHC polypeptide comprises at least 18 residues.

2. The composition of any one of the preceding claims wherein the immunogenic MHC polypeptide does not exceed 50 residues.

3. The composition of any one of the preceding claims wherein the immunogenic MHC polypeptide does not exceed 20 residues.

4. The composition of any one of the preceding claims the immunogenic MHC polypeptide consists of between 18 and 20 residues, inclusive.

5. The composition of claim 4 wherein the immunogenic MHC polypeptide consists of 20 residues.

6. The composition of any one of the preceding claims wherein the immunogenic MHC polypeptide is a homopolymer or a heteropolymer of said sequence from a hypervariable region of an MHC molecule.

7. The composition of any one of the preceding claims wherein the immunogenic MHC polypeptide is linked to a carrier.

8. The composition of any one of the preceding claims wherein the autoimmune disease is multiple sclerosis.

9. The composition of any one of claims 1 to 7, wherein the autoimmune disease is rheumatoid arthritis.

10. The composition of any one of claims 1 to 7, wherein the autoimmune disease is systemic lupus erythematosus, myasthenia gravis, insulin dependent or diabetes mellitus.

11. A pharmaceutical composition comprising a composition according to any one of the preceding claims and a pharmaceutically acceptable excipient.

12. A pharmaceutical composition comprising a composition according to any one of claims 1 to 10 and an adjuvant.

13. A pharmaceutical composition according to claim 11 or claim 12 for use in the prophylaxis or treatment of autoimmune disease.

14. A pharmaceutical composition according to claim 13, wherein the autoimmune disease is systemic lupus erythematosus, multiple sclerosis, myasthenia gravis, rheumatoid arthritis, insulin dependent or diabetes mellitus.

15. A composition in accordance with any one of claims 1 to 10 for use in medical treatment.

16. A pharmaceutical composition according to claim 11 or claim 12 for use in medical treatment.

17. Use of a composition according to any one of claims 1 to 10 in the preparation of a medicament for the prophylaxis or treatment of autoimmune disease.

18. Use of an isolated immunogenic MHC polypeptide wherein the immunogenic MHC polypeptide has a sequence from a hypervariable region of MHC class II molecule associated with an autoimmune disease wherein the hypervariable region is in an MHC class II β chain and wherein the immunogenic MHC polypeptide comprises at least 18 residues, in the preparation of a medicament for the treatment of said autoimmune disease.

## Patentansprüche

1. Zusammensetzung, die ein isoliertes immunogenes MHC-Polypeptid umfasst, worin das immunogene MHC-Polypeptid eine Sequenz aus einer hypervariablen Region eines MHC-Moleküls aufweist, die mit Anfälligkeit für eine Autoimmunerkrankung assoziiert ist, worin die hypervariable Region eine MHC-Klasse II-β-Kette ist und worin das immunogene MHC-Polypeptid zumindest 18 Reste umfasst.

2. Zusammensetzung nach einem der vorangegangenen Ansprüche, worin das immunogene MHC-Polypeptid 50 Reste nicht übersteigt.

3. Zusammensetzung nach einem der vorangegangenen Ansprüche, worin das immunogene MHC-Polypeptid 20 Reste nicht übersteigt.

4. Zusammensetzung nach einem der vorangegangenen Ansprüche, worin das immunogene MHC-Polypeptid aus 18 bis 20 Resten besteht.

5. Zusammensetzung nach Anspruch 4, worin das immunogene MHC-Polypeptid aus 20 Resten besteht.

6. Zusammensetzung nach einem der vorangegangenen Ansprüche, worin das immunogene MHC-Polypeptid ein Homopolymer oder ein Heteropolymer der Sequenz aus einer hypervariablen Region eines MHC-Moleküls ist.

7. Zusammensetzung nach einem der vorangegangenen Ansprüche, worin das immunogene MHC-Polypeptid an einen Träger gebunden ist.

8. Zusammensetzung nach einem der vorangegangenen Ansprüche, worin die Autoimmunerkrankung Multiple Sklerose ist.

9. Zusammensetzung nach einem der Ansprüche 1 bis 7, worin die Autoimmunerkrankung primärchronische Polyarthritis ist.

10. Zusammensetzung nach einem der Ansprüche 1 bis 7, worin die Autoimmunerkrankung systemischer Lupus erythematodes, Myasthenia gravis, Insulin-abhängige oder Diabetes mellitus ist.

11. Pharmazeutische Zusammensetzung, die eine Zusammensetzung nach einem der vorangegangenen Ansprüche und einen pharmazeutisch annehmbaren Exzipienten umfasst.

12. Pharmazeutische Zusammensetzung, die eine Zusammensetzung nach einem der Ansprüche 1 bis 10 und ein Adjuvans umfasst.

13. Pharmazeutische Zusammensetzung nach Anspruch 11 oder 12 zur Verwendung bei der Prophylaxe oder Behandlung einer Autoimmunerkrankung.

14. Pharmazeutische Zusammensetzung nach Anspruch 13, worin die Autoimmunerkrankung systemischer Lupus erythematodes, Multiple Sklerose, Myasthenia gravis, primärchronische Polyarthritis, Insulin-abhängige oder Diabetes mellitus ist.

15. Zusammensetzung nach einem der Ansprüche 1 bis 10 zur Verwendung bei der medizinischen Behandlung.

16. Pharmazeutische Zusammensetzung nach Anspruch 11 oder 12 zur Verwendung bei der medizinischen Behandlung.

17. Verwendung einer Zusammensetzung nach einem der Ansprüche 1 bis 10 zur Herstellung eines Medikaments zur Prophylaxe oder Behandlung einer Autoimmunerkrankung.

18. Verwendung eines isolierten immunogenen MHC-Polypeptids, worin das immunogene MHC-Polypeptid eine Sequenz aus einer hypervariablen Region eines MHC-Klasse II-Moleküls aufweist, die mit einer Autoimmunerkrankung assoziiert ist, worin die hypervariable Region eine MHC-Klasse II-β-Kette ist und worin das immunogene MHC-Polypeptid zumindest 18 Reste umfasst, zur Herstellung eines Medikaments zur Behandlung der Autoimmunerkrankung.

## Revendications

1. Composition comprenant un polypeptide de MHC immunogène isolé où le polypeptide de MHC immunogène a une séquence à partir d'une région hypervariable d'une molécule de MHC associée à la sensibilité à une maladie auto-immune, où la région hypervariable est dans une chaîne β classe II de MHC et où le polypeptide de MHC immunogène comprend au moins 18 résidus.

2. Composition de l'une quelconque des revendications précédentes où le polypeptide de MHC immunogène ne dépasse pas 50 résidus.

3. Composition de l'une quelconque des revendications précédentes où le polypeptide de MHC immunogène ne dépasse pas 20 résidus.

4. Composition de l'une quelconque des revendications précédentes le polypeptide de MHC immunogène consiste en entre 18 et 20 résidus, inclus.

5. Composition de la revendication 4 où le polypeptide de MHC immunogène consiste en 20 résidus.

6. Composition de l'une quelconque des revendications précédentes où le polypeptide de MHC immunogène est un homopolymère ou un hétéropolymère de ladite séquence à partir d'une région hypervariable d'une molécule de MHC.

7. Composition de l'une quelconque des revendications précédentes où le polypeptide de MHC immunogène est enchaîné à un support.

8. Composition de l'une quelconque des revendications précédentes où la maladie auto-immune est la sclérose multiple.

9. Composition de l'une quelconque des revendications 1 à 7, où la maladie auto-immune est l'arthrite rhumatoïde.

10. Composition de l'une quelconque des revendications 1 à 7, où la maladie auto-immune est le lupus érythématoseux généralisé, la myasthenia gravis, le diabète dépendant de l'insuline ou le diabète sucré.

11. Composition pharmaceutique comprenant une composition selon l'une quelconque des revendications précédentes et un excipient pharmaceutiquement acceptable.

12. Composition pharmaceutique comprenant une composition selon l'une quelconque des revendications 1 à 10 et un adjuvant.

13. Composition pharmaceutique selon la revendication 11 ou la revendication 12 à utiliser dans la prophylaxie ou le traitement d'une maladie auto-immune.

14. Composition pharmaceutique selon la revendication 13, où la maladie auto-immune est le lupus érythématoseux généralisé, la sclérose multiple, la myasthenia gravis, l'arthrite rhumatoïde, le diabète dépendant de l'insuline ou le diabète sucré.

15. Composition selon l'une quelconque des revendications 1 à 10 pour une utilisation dans un traitement médical.

16. Composition pharmaceutique selon la revendication 11 ou la revendication 12 pour une utilisation dans un traitement médical.

17. Utilisation d'une composition selon l'une quelconque des revendications 1 à 10 dans la préparation d'un médicament pour la prophylaxie ou le traitement d'une maladie auto-immune.

18. Utilisation d'un polypeptide de MHC immunogène isolé où le polypeptide de MHC immunogène a une séquence d'une région hypervariable de la molécule de MHC classe II associée à une maladie auto-immune, où la région hypervariable est dans une chaîne β de MHC classe II et où le polypeptide de MHC immunogène comprend au moins 18 résidus, dans la préparation d'un médicament pour le traitement de ladite maladie auto-immune.
